# EUROPEAN PATENT APPLICATION

(11) **EP 4 286 533 A1**
(43) Date of publication of application: **06.12.2023**
(21) Application number: 22745728.0
(22) Date of filing: 21.01.2022
(51) Int. Cl.: C12Q 1/686, C08F 130/02, C12N 15/50

(54) **COMPOSITION FOR NUCLEIC ACID AMPLIFICATION**

(30) Priority: 28.01.2021 JP 2021012402
(71) Applicant: NOF Corporation, Shibuya-ku Tokyo 150-6019 (JP)
(72) Inventor: KONDO, Seina, Kawasaki-shi, Kanagawa 210-0865 (JP); SUZUKI, Hirotaka, Kawasaki-shi, Kanagawa 210-0865 (JP); MATSUDA, Masaru, Kawasaki-shi, Kanagawa 210-0865 (JP)
(74) Representative: Weickmann & Weickmann PartmbB
(86) International application number: PCT/JP2022/002103
(87) International publication number: WO 2022/163507

(57) **Abstract**

The present invention provides a composition for nucleic acid amplification containing a polymer containing a constitutional unit derived from 2-(meth)acryloyloxyethyl phosphorylcholine.

## Description

### [Technical Field]

The present invention relates to a composition for nucleic acid amplification.

### [Background Art]

A nucleic acid amplification method is a method for amplifying a target gene, and is a technique used in research in the field of molecular biology, in the field of clinical diagnosis, forensic investigation, and the like. A PCR (Polymerase Chain Reaction) method is known as a nucleic acid amplification method for amplifying a target gene. Since the PCR method can detect nucleic acids with high sensitivity, it is used for diagnosing infectious diseases, detecting viruses, and the like.

The above-mentioned PCR method has been adopted for the detection of the novel coronavirus and is attracting a great deal of attention. According to Non Patent Literature 1, the novel coronavirus (2019-nCoV) is identified by a two-step RT-PCR method that specifically detects two gene regions of the novel coronavirus (2019-nCoV), open reading flame 1a (ORF1a), and spike (S), or genetic testing by the real-time one-step RT-PCR method using TaqMan probes.

With the increase in the number of test specimens due to the epidemic of infectious diseases due to the novel coronavirus, there is a demand for faster disclosure of results. From the aspect of test operability, therefore, it is required to reduce the steps of preparing the PCR reaction liquid. However, from the aspect of storage stability, it was necessary to prepare the composition to be used, immediately before the test. As stabilizers that improve the stability of the aforementioned composition, bovine serum albumin (BSA), gelatin, and non-ionic surfactants are known.

### [Citation List]

### [Non Patent Literature]

[NPL 1]
the National Institute of Infectious Diseases Pathogen Detection Manual 2019-nCoV Ver.2.9.1

### [Summary of Invention]

### [Technical Problem]

The present invention aims to improve storage stability of a composition used in the nucleic acid amplification method (e.g., PCR method).

### [Solution to Problem]

The present inventors have conducted intensive studies and found that the storage stability of a composition used in the nucleic acid amplification method (e.g., PCR method) can be improved using a polymer containing a constitutional unit derived from 2-(meth)acryloyloxyethyl phosphorylcholine. Based on this finding, the present invention provides the following.
[1] A composition for nucleic acid amplification, comprising a polymer comprising a constitutional unit derived from 2-(meth)acryloyloxyethyl phosphorylcholine.
[2] The composition of the aforementioned [1], wherein the aforementioned polymer has a concentration of 0.0001 to 10 w/v%.
[3] The composition of the aforementioned [1] or [2], wherein the composition is used in a PCR method or an RT-PCR method.
[4] The composition of the aforementioned [3], wherein the PCR method or the RT-PCR method is a real-time PCR method or a real-time RT-PCR method.
[5] The composition of the aforementioned [4], wherein the real-time PCR method or the real-time RT-PCR method is a real-time RT-PCR method using a 1-step RT-real-time PCR reagent.
[6] The composition of any one of the aforementioned [1] to [5], wherein the nucleic acid is an RNA derived from an RNA virus.
[7] The composition of the aforementioned [6], wherein the RNA derived from an RNA virus is an RNA derived from SARS-CoV-2.
[8] The composition of any one of the aforementioned [1] to [7], further comprising a primer.
[9] The composition of the aforementioned [8], wherein the primer is an oligonucleotide having a length of 10 to 40 bases.
[10] The composition of the aforementioned [8] or [9], wherein the primer has a concentration of 0.01 to 10 µM.
[11] A method for amplifying a nucleic acid, comprising preparing a reaction liquid for nucleic acid amplification by mixing the composition of any one of the aforementioned [1] to [10] and a sample comprising a nucleic acid to be amplified.
[12] The method of the aforementioned [11], wherein the method is a PCR method or an RT-PCR method.
[13] The method of the aforementioned [12], wherein the method is a real-time PCR method or a real-time RT-PCR method.
[14] The method of the aforementioned [13], wherein the method is a real-time RT-PCR method using a 1-step RT-real-time PCR reagent.
[15] The method of any one of the aforementioned [11] to [14], wherein the nucleic acid is an RNA derived from an RNA virus.
[16] The method of the aforementioned [15], wherein the RNA derived from an RNA virus is an RNA derived from SARS-CoV-2.

### [Advantageous Effects of Invention]

According to the present invention, a composition for nucleic acid amplification which is superior in storage stability can be obtained.

### [Description of Embodiments]

The present invention is described in detail below.

When stepwise numerical ranges are described in the present specification, the lower limit and the upper limit of each numerical range can be combined. In the case of, for example, "preferably 10 to 100, more preferably 20 to 90", the preferred lower limit 10 can be combined with the more preferred upper limit 90 (i.e., the numerical range of "10 to 90" is also within the range of the present specification).

### [Composition for nucleic acid amplification]

The present invention relates to a composition for nucleic acid amplification (hereinafter sometimes to be indicated as "the composition of the present invention"), containing a polymer containing a constitutional unit derived from 2-(meth)acryloyloxyethyl phosphorylcholine (hereinafter sometimes to be indicated as "the polymer of the present invention"). Only one kind of the polymer of the present invention may be used, or two or more kinds thereof may be used in combination. In the following, "2-(meth)acryloyloxyethyl phosphorylcholine" is sometimes indicated as "monomer (1)" and the "constitutional unit derived from monomer (1)" is sometimes indicated as "constitutional unit (1)".

In the present specification, the "2-(meth)acryloyloxyethyl phosphorylcholine" basically means "2-acryloyloxyethyl phosphorylcholine or 2-methacryloyloxyethyl phosphorylcholine". When a plurality of 2-(meth)acryloyloxyethyl phosphorylcholine may be present, the "2-(meth)acryloyloxyethyl phosphorylcholine" means "2-acryloyloxyethyl phosphorylcholine and/or 2-methacryloyloxyethyl phosphorylcholine". Other terms similar to "2-(meth)acryloyloxyethyl phosphorylcholine" mean the same as "(2-(meth)acryloyloxyethyl phosphorylcholine". A commercially available product can be used for 2-(meth)acryloyloxyethyl phosphorylcholine.

In the present specification, the "constitutional unit derived from 2-(meth)acryloyloxyethyl phosphorylcholine" means a constitutional unit having a structure formed by reaction of a carbon-carbon double bond of a (meth)acryloyl group contained in 2-(meth)acryloyloxyethyl phosphorylcholine. Constitutional units derived from other monomers also mean the same as the above.

In the present specification, the "composition for nucleic acid amplification" means a composition used in the nucleic acid amplification method. The nucleic acid amplification method is not particularly limited and examples thereof include Polymerase Chain Reaction (PCR) method, Loop mediated isothermal Amplification (LAMP) method, Transcription Mediated Amplification (TMA) method, Isothermal and Chimeric primer-initiated Amplification of Nucleic acids (IICAN) method, Strand Displacement Amplification (SDA) method, Ligase Chain Reaction (LCR) method, Nucleic Acid Sequence-Based Amplification (NASBA) method, and the like. The nucleic acid amplification method is preferably a PCR method.

The nucleic acid amplification method is preferably a PCR method or a RT-PCR method (reverse transcription PCR method). That is, the composition of the present invention is preferably used in a PCR method or an RT-PCR method. The PCR method or the RT-PCR method is preferably a real-time PCR method or a real-time RT-PCR method, more preferably a real-time RT-PCR method using a 1-step RT-real-time PCR reagent. As used herein, the 1-step RT-real-time PCR reagent means a reagent used for performing a reverse transcription reaction and a PCR reaction continuously or in parallel in one step and measuring the PCR product over time. Examples thereof include a PCR reagent containing a heat-resistant DNA polymerase having reverse transcriptase activity and a fluorescent DNA staining reagent or a fluorescent probe, and the like. The nucleic acid to be amplified is preferably RNA derived from an RNA virus, more preferably RNA derived from SARS-CoV-2.

The polymer of the present invention may be any of a homopolymer consisting of a constitutional unit derived from 2-acryloyloxyethyl phosphorylcholine, a homopolymer consisting of a constitutional unit derived from 2-methacryloyloxyethyl phosphorylcholine, and a copolymer consisting of a constitutional unit derived from 2-acryloyloxyethyl phosphorylcholine and a constitutional unit derived from 2-methacryloyloxyethyl phosphorylcholine. The aforementioned copolymer may be a random copolymer, a block copolymer, or a copolymer containing both a random portion and a block portion.

When the polymer of the present invention does not contain constitutional units other than the constitutional unit (1), the polymer of the present invention is preferably a homopolymer consisting of a constitutional unit derived from 2-(meth)acryloyloxyethyl phosphorylcholine (i.e., homopolymer consisting of a constitutional unit derived from 2-acryloyloxyethyl phosphorylcholine or homopolymer consisting of a constitutional unit derived from 2-methacryloyloxyethyl phosphorylcholine), more preferably a homopolymer consisting of a constitutional unit derived from 2-methacryloyloxyethyl phosphorylcholine.

The polymer of the present invention may be a copolymer containing other constitutional unit derived from other monomers different from monomer (1) in addition to the constitutional unit (constitutional unit (1)) derived from 2-(meth)acryloyloxyethyl phosphorylcholine (monomer (1)). Only one kind of other monomer may be used, or two or more kinds thereof may be used in combination. The aforementioned copolymer may be a random copolymer, a block copolymer, or a copolymer containing both a random portion and a block portion.

Examples of other constitutional unit include a constitutional unit (hereinafter sometimes to be indicated as "constitutional unit (2)") derived from alkyl (meth)acrylate free of a hydroxy group (hereinafter sometimes to be indicated as "monomer (2)"). The alkyl of the alkyl (meth)acrylate may have a substituent other than a hydroxy group. Examples of the aforementioned substituent include halogen atom, epoxy group, alkoxy groups such as methoxy group and the like, aryl groups such as phenyl group and the like, aryloxy groups such as phenoxy group and the like, heterocyclic groups such as tetrahydrofuranyl group and the like, and the like. Only one kind of monomer (2) may be used, or two or more kinds thereof may be used in combination. That is, the polymer of the present invention may contain one or more kinds of constitutional unit (2).

Examples of monomer (2) include methyl (meth)acrylate, ethyl (meth)acrylate, propyl (meth)acrylate, butyl (meth)acrylate, pentyl (meth)acrylate, hexyl (meth)acrylate, heptyl (meth)acrylate, octyl (meth)acrylate, nonyl (meth)acrylate, decyl (meth)acrylate, undecyl (meth)acrylate, dodecyl (meth)acrylate, tridecyl (meth)acrylate, tetradecyl (meth)acrylate, pentadecyl (meth)acrylate, cetyl (meth)acrylate, heptadecyl (meth)acrylate, stearyl (meth)acrylate, benzyl (meth)acrylate, phenoxyethyl (meth)acrylate, tetrahydrofurfuryl (meth)acrylate, glycidyl (meth)acrylate, and the like. Among these, butyl (meth)acrylate, stearyl (meth)acrylate, and benzyl (meth)acrylate are preferred, butyl methacrylate, stearyl methacrylate, and benzyl methacrylate are more preferred. A commercially available product can be used for monomer (2).

When the polymer of the present invention contains constitutional unit (1) and constitutional unit (2), from the aspect of stabilizing action, the amount of constitutional unit (1) (i.e., monomer (1) used in polymerization) is preferably 5 to 95 mol, more preferably 20 to 80 mol, further preferably 60 to 80 mol, and the amount of constitutional unit (2) (i.e., monomer (2) used in polymerization) is preferably 5 to 95 mol, more preferably 20 to 80 mol, further preferably 20 to 40 mol, each with respect to the total 100 mol of the constitutional unit (1) and constitutional unit (2) (i.e., total 100 mol of monomer (1) and monomer (2) used in polymerization).

Examples of other constitutional unit include a constitutional unit (hereinafter sometimes to be abbreviated as "constitutional unit (3)") derived from a hydroxy group-containing alkyl (meth)acrylate (hereinafter sometimes to be abbreviated as "monomer (3)"). Only one kind of monomer (3) may be used, or two or more kinds thereof may be used in combination. That is, the polymer of the present invention may contain one or more kinds of constitutional unit (3).

The number of hydroxy groups in monomer (3) is preferably not less than 2, more preferably 2 to 5. Examples of monomer (3) include, glycerol mono(meth)acrylate, threitol mono(meth)acrylate, erythritol mono(meth)acrylate, xylitol mono(meth)acrylate, arabitol mono(meth)acrylate, mannitol mono(meth)acrylate, galactitol mono(meth)acrylate, sorbitol mono(meth)acrylate, and the like. Among these, glycerol mono(meth)acrylate is preferred, and glycerol monomethacrylate is more preferred.

A commercially available product can be used for monomer (3), or may be produced by a known method. For example, monomer (3) can be produced by an esterification reaction of (meth)acrylic acid or a derivative thereof (e.g., acid chloride) with a polyhydric alcohol having 3 or more hydroxy groups. Esterification reaction is well known, those of ordinary skill in the art can set the conditions appropriately and perform the reaction.

When the polymer of the present invention contains constitutional unit (1) and constitutional unit (3), from the aspect of stabilizing action, the amount of constitutional unit (1) (i.e., monomer (1) used in polymerization) is preferably 5 to 95 mol, more preferably 20 to 80 mol, and the amount of constitutional unit (3) (i.e., monomer (3) used in polymerization) is preferably 5 to 95 mol, more preferably 20 to 80 mol, each with respect to the total 100 mol of the constitutional unit (1) and constitutional unit (3) (i.e., total 100 mol of monomer (1) and monomer (3) used in polymerization).

When the polymer of the present invention contains constitutional unit (1), constitutional unit (2), and constitutional unit (3), from the aspect of stabilizing action, the amount of constitutional unit (1) (i.e., monomer (1) used in polymerization) is preferably 5 to 90 mol, more preferably 10 to 80 mol, further preferably 30 to 50 mol the amount of constitutional unit (2) (i.e., monomer (2) used in polymerization) is preferably 5 to 90 mol, more preferably 10 to 80 mol, further preferably 30 to 50 mol, and the amount of constitutional unit (3) (i.e., monomer (3) used in polymerization) is preferably 5 to 90 mol, more preferably 10 to 80 mol, further preferably 10 to 40 mol, each with respect to the total 100 mol of the constitutional unit (1), constitutional unit (2), and constitutional unit (3) (i.e., total 100 mol of monomer (1), monomer (2), and monomer (3) used in polymerization).

The polymer of the present invention may contain a constitutional unit (hereinafter sometimes to be indicated as "constitutional unit (4)") derived from a monomer different from the aforementioned constitutional unit (1), constitutional unit (2), or constitutional unit (3) (hereinafter sometimes to be indicated as "monomer (4)"). Only one kind of monomer (4) may be used, or two or more kinds thereof may be used in combination. That is, the polymer of the present invention may contain one or more kinds of constitutional unit (4).

Examples of monomer (4) include alkenyl (meth)acrylates such as allyl (meth)acrylate and the like; cycloalkyl (meth)acrylates such as cyclohexyl (meth)acrylate and the like; esters of (meth)acrylic acid and monoterpene alcohol such as isobornyl (meth)acrylate and the like; styrene monomers such as styrene, methylstyrene, chloromethylstyrene, and the like; vinyl ether monomers such as methyl vinyl ether, butyl vinyl ether, and the like; vinyl ester monomers such as vinyl acetate, vinyl propionate, and the like, and the like.

The amount of constitutional unit (4) in the polymer of the present invention is preferably not more than 40 mol%, more preferably not more than 20 mol% with respect to the total constitutional units. More preferably, the polymer of the present invention does not contain constitutional unit (4).

The polymer of the present invention is preferably at least one selected from the group consisting of a homopolymer consisting of one kind of constitutional unit (1), a copolymer consisting of constitutional unit (1) and constitutional unit (2), and a copolymer consisting of constitutional unit (1), constitutional unit (2), and constitutional unit (3). More preferably, it is a homopolymer consisting of one kind of constitutional unit (1), a copolymer consisting of constitutional unit (1) and constitutional unit (2), or a copolymer consisting of constitutional unit (1), constitutional unit (2), and constitutional unit (3). In the present specification, the "homopolymer consisting of one kind of constitutional unit (1)" means a homopolymer whose all constitutional units (repeating units) consist of one kind of constitutional unit (1), the "copolymer consisting of constitutional unit (1) and constitutional unit (2)" means a copolymer whose all constitutional units (repeating units) consist of constitutional unit (1) and constitutional unit (2), and the "copolymer consisting of constitutional unit (1), constitutional unit (2), and constitutional unit (3)" means a copolymer whose all constitutional units (repeating units) consist of constitutional unit (1), constitutional unit (2), and constitutional unit (3). Other similar expressions mean the same.

While the weight average molecular weight of the polymer of the present invention is not particularly limited, it is preferably 5,000 to 2,000,000, more preferably 10,000 to 1,500,000. The weight average molecular weight can be determined based on polyethylene glycol by gel filtration chromatography using, for example, EcoSEC system (manufactured by Tosoh Corporation) or the like.

The copolymer of the present invention can be produced by known methods (e.g., the method described in WO 2018/216628).

The concentration of the polymer of the present invention in the composition for nucleic acid amplification is preferably not less than 0.0001 w/v%, more preferably not less than 0.0005 w/v%, further preferably not less than 0.0010 w/v%, particularly preferably not less than 0.010 w/v%, most preferably not less than 0.10 w/v%, and preferably not more than 10 w/v%, more preferably not more than 5 w/v%, further preferably not more than 1.0 w/v%, from the aspects of the stability action. When two or more kinds of the polymer of the present invention are used, the aforementioned concentration means the total concentration of the two or more kinds of the polymer of the present invention. When two or more kinds of the following other components are used, the concentrations or amounts described for such other components also refer to the total of the concentrations or amounts of the two or more components.

### <Examples of preferred polymer of the present invention>

Examples of the preferred polymer of the present invention include the polymer (I) of the present invention and the polymer (II) of the present invention below.

### (Polymer (I) of the present invention)

The polymer (I) of the present invention is at least one selected from the group consisting of the following homopolymer (I-1), copolymer (I-2), and copolymer (I-3), preferably the following homopolymer (I-1), copolymer (I-2), or copolymer (I-3) :
homopolymer (I-1) consisting of constitutional unit (1) derived from 2-(meth)acryloyloxyethyl phosphorylcholine;
copolymer (I-2) consisting of constitutional unit (1) derived from 2-(meth)acryloyloxyethyl phosphorylcholine, and constitutional unit (2) derived from butyl (meth)acrylate, stearyl (meth)acrylate, or benzyl (meth)acrylate, in which
the amount of constitutional unit (1) is 5 to 95 mol and the amount of constitutional unit (2) is 5 to 95 mol each with respect to the total 100 mol of constitutional unit (1) and constitutional unit (2); and
copolymer (I-3) consisting of constitutional unit (1) derived from 2-(meth)acryloyloxyethyl phosphorylcholine, constitutional unit (2) derived from butyl (meth)acrylate, stearyl (meth)acrylate, or benzyl (meth)acrylate, and constitutional unit (3) derived from glycerol mono(meth)acrylate, in which
the amount of constitutional unit (1) is 5 to 90 mol, the amount of constitutional unit (2) is 5 to 90 mol, and the amount of constitutional unit (3) is 5 to 90 mol each with respect to the total 100 mol of constitutional unit (1), constitutional unit (2), and constitutional unit (3).

In the polymer (I) of the present invention, it is preferred that the amount of constitutional unit (1) in copolymer (I-2) is 20 to 80 mol, and the amount of constitutional unit (2) therein is 20 to 80 mol, and the amount of constitutional unit (1) in copolymer (I-3) is 10 to 80 mol, the amount of constitutional unit (2) therein is 10 to 80 mol, and the amount of constitutional unit (3) therein is 10 to 80 mol. The standard for the amounts of the aforementioned constitutional units in copolymer (I-2) is a total 100 mol of constitutional unit (1) and constitutional unit (2). The standard for the amounts of the aforementioned constitutional units in copolymer (I-3) is a total 100 mol of constitutional unit (1), constitutional unit (2), and constitutional unit (3).

In the polymer (I) of the present invention, it is more preferred that the amount of constitutional unit (1) in copolymer (I-2) is 60 to 80 mol, and the amount of constitutional unit (2) therein is 20 to 40 mol, and the amount of constitutional unit (1) in copolymer (I-3) is 30 to 50 mol, the amount of constitutional unit (2) therein is 30 to 50 mol, and the amount of constitutional unit (3) therein is 10 to 40 mol. The standard for the amounts of the aforementioned constitutional units in copolymer (I-2) is a total 100 mol of constitutional unit (1) and constitutional unit (2). The standard for the amounts of the aforementioned constitutional units in copolymer (I-3) is a total 100 mol of constitutional unit (1), constitutional unit (2), and constitutional unit (3).

### (Polymer (II) of the present invention)

The polymer (II) of the present invention is at least one selected from the group consisting of the following homopolymer (II-1), copolymer (II-2), and copolymer (II-3), preferably the following homopolymer (II-1), copolymer (II-2), or copolymer (II-3) :
homopolymer (II-1) consisting of constitutional unit (1) derived from methacryloyloxyethyl phosphorylcholine;
copolymer (II-2) consisting of constitutional unit (1) derived from methacryloyloxyethyl phosphorylcholine, and constitutional unit (2) derived from butyl methacrylate, stearyl methacrylate, or benzyl methacrylate, in which
the amount of constitutional unit (1) is 20 to 80 mol and the amount of constitutional unit (2) is 20 to 80 mol each with respect to the total 100 mol of constitutional unit (1) and constitutional unit (2); and
copolymer (II-3) consisting of constitutional unit (1) derived from methacryloyloxyethyl phosphorylcholine, constitutional unit (2) derived from butyl methacrylate, stearyl methacrylate, or benzyl methacrylate, and constitutional unit (3) derived from glycerol monomethacrylate, in which
the amount of constitutional unit (1) is 10 to 80 mol, the amount of constitutional unit (2) is 10 to 80 mol, and the amount of constitutional unit (3) is 10 to 80 mol each with respect to the total 100 mol of constitutional unit (1), constitutional unit (2), and constitutional unit (3).

In the polymer (II) of the present invention, it is preferred that the amount of constitutional unit (1) in copolymer (II-2) is 60 to 80 mol, and the amount of constitutional unit (2) therein is 20 to 40 mol, and the amount of constitutional unit (1) in copolymer (II-3) is 30 to 50 mol, the amount of constitutional unit (2) therein is 30 to 50 mol, and the amount of constitutional unit (3) therein is 10 to 40 mol. The standard for the amounts of the aforementioned constitutional units in copolymer (II-2) is a total 100 mol of constitutional unit (1) and constitutional unit (2). The standard for the amounts of the aforementioned constitutional units in copolymer (II-3) is a total 100 mol of constitutional unit (1), constitutional unit (2), and constitutional unit (3).

### <Other component>

The composition of the present invention can be prepared by dissolving the aforementioned polymer in a solvent such as water, together with, where necessary, other components used for nucleic acid amplification. That is, the composition of the invention is preferably a composition containing the aforementioned polymer and water (and other components where necessary).

As other components used for nucleic acid amplification, known components used for known nucleic acid amplification represented by PCR can be used. Examples of such component include pH buffer, metal salt, nucleic acid synthase, primer, dideoxynucleoside triphosphate (dNTP), and the like. Only one kind of other component may be used, or two or more kinds thereof may be used in combination.

Examples of the pH buffer include EDTA (ethylenediaminetetraacetic acid) solution, Tris-HCL (tris(hydroxymethyl)aminomethane-hydrochloric acid) solution, TAE (tris-acetic acid-EDTA) solution, and TE (tris-EDTA buffer) solution.

Examples of the metal salt include magnesium chloride, manganese(II) acetate, sodium sulfate, sodium dodecylsulfate, and the like.

Examples of the nucleic acid synthase include DNA polymerase, RNA polymerase, reverse transcriptase, and the like.

Examples of the primer include oligonucleotides with a length of 10 to 40 bases. The length of aforementioned oligonucleotide is preferably 15 to 30 bases, more preferably 17 to 25 bases. The aforementioned oligonucleotide can be designed and prepared by known methods. The aforementioned oligonucleotides may have a group that emits fluorescence and formed from fluorescein or the like.

Only one kind of primer may be used, or two kinds thereof may be used in a pair. A plurality of primers may also be used to simultaneously amplify plural regions. The concentration of the primer in the composition of the present invention is preferably 0.01 to 10 µM, more preferably 0.05 to 5.0 µM, further preferably 0.1 to 3.0 µM.

Examples of the dideoxynucleoside triphosphate (dNTP) include deoxyadenosine triphosphate (dATP), deoxyguanosine triphosphate (dGTP), deoxythymidine triphosphate (dTTP), and deoxycytidine triphosphate (dCTP).

Examples of other further component include bovine serum albumin, dimethyl sulfoxide, dimethylformamide, betaine, formamide, gelatin, glycerol, polyethylene glycol, spermidine, T4 Gane 32 protein, 4-(1,1,3,3-tetramethylbutyl)phenyl-polyethylene glycol, tert-octylphenoxypolyethoxyethanol, polyethylene glycol tert-octylphenyl ether, urea, and the like.

### [Nucleic acid amplification method]

The present invention also provides a method for amplifying a nucleic acid, including preparing a reaction liquid for nucleic acid amplification by mixing the composition of the present invention and a sample comprising a nucleic acid to be amplified. The polymer of the present invention and the composition of the present invention in the nucleic acid amplification method of the present invention are as described above. In addition, the nucleic acid amplification method is as described above unless particularly described.

The sample used in the nucleic acid amplification method of the present invention contains nucleic acids to be amplified. The sample is preferably a sample solution containing water and nucleic acids to be amplified. The sample may contain one kind of nucleic acid, or may contain two or more kinds of nucleic acid. The concentration of the nucleic acid in the sample is appropriately determined according to the purpose of nucleic acid amplification. When concentration preparation is possible, it is preferably 1 to 10²⁰ copy/µL, more preferably 1 to 10¹⁰ copy/µL, further preferably 1 to 10⁵ copy/µL, particularly preferably 1 to 500 copy/µL, most preferably 1 to 100 copy/µL. The nucleic acid to be amplified is preferably an RNA derived from an RNA virus, more preferably an RNA derived from SARS-CoV-2.

In the diffusion amplification method of the present invention, the amount of a sample used is preferably a trace amount to 1 µL, more preferably 0.01 to 1 µL, further preferably 0.2 to 0.4 µL, per 1 µL of the amount of the composition of the present invention used.

The nucleic acid amplification method of the present invention is preferably a PCR method or an RT-PCR method, more preferably a real-time PCR method or a real-time RT-PCR method, further preferably a real-time RT-PCR method using a 1-step RT-real-time PCR reagent.

### [Example]

The present invention is explained in more detail in the following by referring to Examples and the like, which are not to be construed as limitative.

### [Synthesis of polymer]

### [Synthetic Example 1]

As monomer (1), 40.0 g of 2-methacryloyloxyethylphosphorylcholine (hereinafter sometimes to be abbreviated as "MPC") was weighed into a glass flask for polymerization, 60.0 g of purified water was added to dissolve monomer (1), and 0.31 g of azobisisobutyronitrile was added as a polymerization initiator to the obtained solution. After the inside of the reaction container was sufficiently replaced with nitrogen, polymerization was performed by heating at 70°C for 6 hr while stirring. The obtained reaction solution was ice-cooled and added dropwise to diethyl ether to precipitate a polymer. The precipitate was collected by filtration, washed with diethyl ether, and vacuum dried to give a homopolymer (hereinafter to be indicated as "polymer 1") as a white powder. The weight average molecular weight of polymer 1 was 1,030,000 based on polyethylene glycol as measured by gel filtration chromatography (hereinafter sometimes to be abbreviated as "GPC") under the below-mentioned conditions.

### [Synthetic Example 2]

In the same manner as in Synthetic Example 1 except that MPC as monomer (1) and butyl methacrylate (hereinafter sometimes to be abbreviated as "BMA") as monomer (2) were charged in a reaction container at monomer (1)/monomer (2)=80/20 (molar ratio), a random copolymer (hereinafter to be indicated as "polymer 2") was obtained. The weight average molecular weight of polymer 2 was 600,000 based on polyethylene glycol as measured by GPC under the below-mentioned conditions.

### [Synthetic Example 3]

In the same manner as in Synthetic Example 1 except that MPC as monomer (1), BMA as monomer (2), and glycerol monomethacrylate (hereinafter sometimes to be abbreviated as "GLM") as monomer (3) were charged in a reaction container at monomer (1)/monomer (2)/monomer (3)=40/40/20 (molar ratio), a random copolymer (hereinafter to be indicated as "polymer 3") was obtained. The weight average molecular weight of polymer 3 was 22,000 based on polyethylene glycol as measured by GPC under the below-mentioned conditions.

### [Synthetic Example 4]

In the same manner as in Synthetic Example 1 except that MPC as monomer (1) and stearyl methacrylate (hereinafter sometimes to be abbreviated as "SMA") as monomer (2) were charged in a reaction container at monomer (1)/monomer (2)=80/20 (molar ratio), a random copolymer (hereinafter to be indicated as "polymer 4") was obtained. The weight average molecular weight of polymer 4 was 43,000 based on polyethylene glycol as measured by GPC under the below-mentioned conditions.

### [Synthetic Example 5]

In the same manner as in Synthetic Example 1 except that MPC as monomer (1) and benzyl methacrylate (hereinafter sometimes to be abbreviated as "BNM") as monomer (2) were charged in a reaction container at monomer (1)/monomer (2)=80/20 (molar ratio), a copolymer (hereinafter to be indicated as "polymer 5") was obtained. The weight average molecular weight of polymer 5 was 240,000 based on polyethylene glycol as measured by GPC under the below-mentioned conditions.

### [GPC measurement]

GPC measurement of the polymers 1 to 5 obtained in Synthetic Examples 1 to 5 was performed under the following conditions.
GPC system: EcoSEC system (manufactured by Tosoh Corporation)
column: Shodex OHpak SB-802.5HQ (manufactured by Showa Denko K.K.), and SB-806HQ (manufactured by Showa Denko K.K.) connected in tandem
eluent: 20 mM sodium phosphate buffer (pH 7.4)
detector: differential refractive index detector
molecular weight standard: EasiVial PEG/PEO (manufactured by Agilent Technologies)
flow rate: 0.5 mL/min
column temperature: 40°C
sample: obtained polymer diluted with eluent to final concentration of 0.1 wt%
injection volume: 100 µL

The monomers used in Synthetic Examples 1 to 5 and molar ratios thereof, and weight average molecular weights of the obtained polymers are summarized in the following Table 1.

**[Table 1]**

| | monomer (1) | monomer (2) | monomer (3) | molar ratio of monomers | weight average molecular weight |
|---|---|---|---|---|---|
| polymer 1 | MPC | - | - | 100/0/0 | 1,030,000 |
| polymer 2 | MPC | BMA | - | 80/20/0 | 600,000 |
| polymer 3 | MPC | BMA | GLM | 40/40/20 | 22,000 |
| polymer 4 | MPC | SMA | - | 80/20/0 | 43,000 |
| polymer 5 | MPC | BNM | - | 80/20/0 | 240,000 |

| | | | | | |
|---|---|---|---|---|---|
| molar ratio of monomers = monomer (1)/monomer (2)/monomer (3) MPC: 2-methacryloyloxyethyl phosphorylcholine BMA: butyl methacrylate SMA: stearyl methacrylate BNM: benzyl methacrylate GLM: glycerol monomethacrylate | | | | | |

### [Examples 1 to 5]

Using SARS-CoV-2 RT-qPCR Detection Kit (manufactured by FUJIFILM Wako Pure Chemical Corporation) and the following amounts to be added, a solution prior to the addition of polymers (hereinafter "solution A") was prepared. The constitution of solution A is as shown in the following Table 2.

**[Table 2]**

| reagent name | amount added |
|---|---|
| 5x Reaction Buffer | 4 µL |
| 4.2 mmol/L dNTPs | 4 µL |
| 50 mmol/L Manganese(II) Acetate | 1 µL |
| Hot Start Reverse Transcription DNA Polymerase | 0.25 µL |
| Distilled Water, Nuclease-free | 0.55 µL |
| Fw Primer NIID 2019-nCOV N F2 | 1 µL |
| Rv Primer NIID 2019-nCOV N R2 | 1.4 µL |
| TaqMan Probe NIID 2019-nCOV N P2 | 0.8 µL |
| total | 13 µL |

| | |
|---|---|
| reagent: components of SARS-CoV-2 RT-qPCR Detection Kit (manufactured by FUJIFILM Wako Pure Chemical Corporation) amount added: amount added per well | |

In Examples 1 to 5, 2 µL of aqueous solutions of 0.5 w/v% polymers 1 to 5 were added to each well of solution A having the composition shown in Table 2 to prepare 15 µL of the compositions for nucleic acid amplification (concentration of each polymer in the compositions: 0.067 w/v%).

A sample solution having a nucleic acid concentration of 10 copy/µL was prepared using Positive Control RNA, N set No.2 (N2) attached to the aforementioned kit. The obtained sample solution was added to the composition for nucleic acid amplification in an amount of 5 µL per well to prepare a PCR reaction liquid (concentration of each polymer in the PCR reaction liquid: 0.050 w/v%). A PCR method was performed using a qPCR instrument, StepOnePlus^{™} Real-Time PCR System (manufactured by Applied Biosystems, and fluorescence intensity at the endpoint of the PCR reaction liquid was measured. The temperature program is as shown in the following Table 3.

**[Table 3]**

| step | reaction conditions | |
|---|---|---|
| 1 | 90°C, 30 sec | |
| 2 | 60°C, 5 min | |
| 3 | 95°C, 1 min | |
| 4 | 95°C, 3 sec | 60 cycles |
| 5 | 60°C, 10 sec | |
| 6 | 4°C | |

In the same manner as above except that the aforementioned sample solution was added to the composition for nucleic acid amplification after incubation of the composition for nucleic acid amplification at 35°C for 2 hr, the fluorescence intensity at the endpoint of the PCR reaction liquid was measured.

Using mean fluorescence intensity (measured three times) obtained using a composition for nucleic acid amplification that was not incubated (hereinafter indicated as "mean fluorescence intensity before incubation) and mean fluorescence intensity (measured three times) obtained using an incubated composition for nucleic acid amplification (hereinafter indicated as "mean fluorescence intensity after incubation), the fluorescence intensity ratio was calculated from the following formula:
fluorescence intensity ratio = mean fluorescence intensity after incubation/mean fluorescence intensity before incubation. The results are shown in the following Table 4.

### [Comparative Example 1]

In Comparative Example 1, the fluorescence intensity ratio was calculated in the same manner as in Examples 1 to 5 except that a composition obtained by adding Distilled Water, Nuclease-free (2 µL per well) to solution A having the composition shown in Table 2 was used as the composition for nucleic acid amplification. The results are shown in the following Table 4.

**[Table 4]**

| | polymer used | fluorescence intensity ratio |
|---|---|---|
| Comparative Example 1 | - | 0.14 |
| Example 1 | polymer 1 | 0.34 |
| Example 2 | polymer 2 | 0.16 |
| Example 3 | polymer 3 | 0.54 |
| Example 4 | polymer 4 | 0.74 |
| Example 5 | polymer 5 | 0.34 |

As shown in Table 4, Examples 1 to 5 using polymers 1 to 5 showed higher fluorescence intensity ratios than Comparative Example 1 not using the aforementioned polymer. From these results, it is clear that a composition for nucleic acid amplification containing a polymer containing a constitutional unit derived from 2-(meth)acryloyloxyethyl phosphorylcholine is superior in storage stability.

### [Examples 6 to 8]

In Examples 6 to 8, the fluorescence intensity ratio was calculated in the same manner as in Examples 1 to 5 except that aqueous solutions of 5 w/v% polymers 1 to 3 were added (2 µL per well) to solution A having the composition shown in Table 2 to prepare 15 µL of compositions for nucleic acid amplification (concentration of each polymer in composition: 0.67 w/v%, concentration of each polymer in PCR reaction liquid: 0.50 w/v%). The results are shown in the following Table 5.

**[Table 5]**

| | polymer used | fluorescence intensity ratio |
|---|---|---|
| Example 6 | polymer 1 | 0.38 |
| Example 7 | polymer 2 | 0.87 |
| Example 8 | polymer 3 | 0.76 |

As shown in Tables 4 and 5, Examples 6 to 8 using polymers 1 to 3 showed higher fluorescence intensity ratios than Comparative Example 1 not using the aforementioned polymer. From these results, it is clear that a composition for nucleic acid amplification containing a polymer containing a constitutional unit derived from 2-(meth)acryloyloxyethyl phosphorylcholine is superior in storage stability.

### [Example 9]

In Example 9, aqueous solution of 0.5 w/v% polymer 1 was added (2 µL per well) to solution A having the composition shown in Table 2 to prepare 15 µL of a composition for nucleic acid amplification (concentration of polymer 1 in composition: 0.067 w/v%). This composition for nucleic acid amplification was incubated at 4°C for 1 and 3 months to prepare sample solutions with a nucleic acid concentration of 40 copy/µL, and the fluorescence intensity ratio after incubation for one month and the fluorescence intensity ratio after incubation for 3 months were calculated in the same manner as in Examples 1 to 5 (concentration of polymer 1 in PCR reaction liquid: 0.050 w/v%). The results are shown in the following Table 6.

### [Comparative Example 2]

In Comparative Example 2, the fluorescence intensity ratio after incubation for one month and the fluorescence intensity ratio after incubation for 3 months were calculated in the same manner as in Example 9 except that a composition obtained by adding Distilled Water, Nuclease-free (2 µL per well) to solution A having the composition shown in Table 2 was used as the composition for nucleic acid amplification. The results are shown in the following Table 6.

**[Table 6]**

| | polymer used | fluorescence intensity ratio | |
|---|---|---|---|
| | | after incubation for one month at 4°C | after incubation for 3 months at 4°C |
| Comparative Example 2 | - | 0.80 | 0.28 |
| Example 9 | polymer 1 | 1.00 | 0.78 |

As shown in Table 6, Example 9 using polymer 1 showed a higher fluorescence intensity ratio than Comparative Example 2 not using the aforementioned polymer. From these results, it is clear that a composition for nucleic acid amplification containing a polymer containing a constitutional unit derived from 2-(meth)acryloyloxyethyl phosphorylcholine is superior in storage stability.

### [Industrial Applicability]

The composition of the present invention is superior in storage stability. The composition of the present invention can be preferably used in nucleic acid amplification methods (particularly PCR method) for genetic testing, microbial testing, viral testing, and the like.

This application is based on a patent application No. 2021-12402 filed in Japan, the contents of which are incorporated in full herein.

## Claims

1. A composition for nucleic acid amplification, comprising a polymer comprising a constitutional unit derived from 2-(meth)acryloyloxyethyl phosphorylcholine.

2. The composition according to claim 1, wherein the polymer has a concentration of 0.0001 to 10 w/v%.

3. The composition according to claim 1 or 2, wherein the composition is used in a PCR method or an RT-PCR method.

4. The composition according to claim 3, wherein the PCR method or the RT-PCR method is a real-time PCR method or a real-time RT-PCR method.

5. The composition according to claim 4, wherein the real-time PCR method or the real-time RT-PCR method is a real-time RT-PCR method using a 1-step RT-real-time PCR reagent.

6. The composition according to any one of claims 1 to 5, wherein the nucleic acid is an RNA derived from an RNA virus.

7. The composition according to claim 6, wherein the RNA derived from an RNA virus is an RNA derived from SARS-CoV-2.
